# EUROPÄISCHE PATENTSCHRIFT

(11) **EP 0 977 721 B1**
(45) Veröffentlichungstag und Bekanntmachung des Hinweises auf die Patenterteilung: **27.03.2002**
(21) Anmeldenummer: 98928204.1
(22) Anmeldetag: 17.04.1998
(51) Int. Cl.: C07C 43/13, C07C 41/05, B01J 29/00

(54) **EINSTUFIGES VERFAHREN ZUR HERSTELLUNG VON GLYKOLMONOETHERN AUS OLEFINEN**
SINGLE-STEP METHOD FOR PRODUCING GLYCOL MONOETHERS FROM OLEFINS
PROCEDE A UNE ETAPE POUR PREPARER DES MONOETHERS GLYCOLIQUES A PARTIR D'OLEFINES

(30) Priorität: 24.04.1997 DE 19717320
(43) Veröffentlichungstag der Anmeldung: 09.02.2000
(73) Patentinhaber: BASF AKTIENGESELLSCHAFT, 67056 Ludwigshafen (DE)
(72) Erfinder: MÜLLER, Ulrich, D-67435 Neustadt (DE); GROSCH, Georg, Heinrich, D-67098 Bad Dürkheim (DE); WALCH, Andreas, D-69120 Heidelberg (DE); RIEBER, Norbert, D-68259 Mannheim (DE)
(86) Internationale Anmeldenummer: EP9802281
(87) Internationale Veröffentlichungsnummer: WO9847845

(56) Entgegenhaltungen:
- EP-A- 0 100 118
- EP-A- 0 266 825
- DE-A- 1 924 672
- DE-B- 1 276 621
- GB-A- 2 252 556

## Beschreibung

Die vorliegende Erfindung betrifft ein verbessertes Verfahren zur Herstellung von Glykolmonoethern aus Olefinen. Weiterhin betrifft die Erfindung eine Katalysatormischung, welche bei dem erfindungsgemäßen Verfahren zum Einsatz gelangt.

Glykolmonoether finden breite technische Anwendung als Lösungsmittel, Absorptionsflüssigkeiten in der Gaswäsche, Frostschutzmittel, Hydraulikflüssigkeiten, Gleitmittel, Weichmacher, Tenside, Vorstufen für Faserprodukte wie etwa Polyester oder Urethane, Zusatzstoffe für Druckfarben sowie in kosmetischen Produkten und Körperpflegemitteln.

Wichtigste Produkte sind die entsprechenden Glykolether des Ethylens und Propens. Gewöhnlicherweise stellt man diese Glykolether durch Umsetzen von Epoxiden der zugrundeliegenden Olefine mit den entsprechenden Alkoholen her.

Nachteilig bei dieser Vorgehensweise ist, daß man mehrstufig zunächst aus den Olefinen die Epoxide herstellen muß, um danach bei höherer Temperatur mit z.B. Schwefelsäure die Epoxide ringöffnend mit Alkoholen zur Reaktion zu bringen.

Aufgabe der vorliegenden Erfindung war es, ein einfacheres Herstellverfahren für Glykolmonoether bereitzustellen. Überraschenderweise wurde nunmehr festgestellt, daß man die vorgenannten Nachteile überwinden kann, wenn man in einer einfachen einstufigen Synthese Olefine mit einem üblichen Epoxidierungsreagenz an geeigneten Epoxidierungskatalysatoren umsetzt und gleichzeitig die Anwesenheit von hydroxylgruppenhaltigen organischen Verbindungen wie Alkoholen und sauren oder basischen festen, heterogenen Alkoxylierungskatalysatoren zuläßt. Dabei werden die intermediär gebildeten Epoxide in situ an den zugesetzten festen, heterogenen Alkoxylierungskatalysatoren zu Glykolmonoethern umgesetzt.

Demgemäß wurde ein Verfahren zur Herstellung von Glykolmonoethern aus Olefinen gefunden, welches dadurch gekennzeichnet ist, daß man die Olefine mit einem Epoxidierungsreagenz bei gleichzeitiger Anwesenheit von hydroxylgruppenhaltigen organischen Verbindungen an einer Mischung aus Epoxidierungskatalysatoren und festen heterogenen Alkoxylierungskatalysatoren umsetzt.

Als Epoxidierungskatalysatoren in der Katalysatormischung kommen vorzugsweise titanhaltige Silikate oder titan-, vanadium-, germanium- oder zinnhaltige Zeolithe in Betracht, insbesondere Titan- oder Vanadiumsilikalite mit Zeolith-Struktur mit röntgenographischer Zuordnung zur MFI-, MEL-, BEA-, MTW-, TON-, FER- oder MFI/MEL-Mischstruktur. Derartige Epoxidierungskatalysatoren sind beispielsweise beschrieben in der DE-A 44 25 672, Die genannten Titan- oder Vanadiumsilikalite können gemäß DE-A 44 25 672 Edelmetall wie Platinmetalle in Mengen von 0,01 bis 20 Gew. -% enthalten, insbesondere ist dies bei Verwendung einer Wasserstoff-Sauerstoff-Mischung als Epoxidierungsreagenz vorteilhaft. GB-A-2 252 556 beschreibt ein Verfahren zur Herstellung von Glykolmonoethern aus Olefinen unter Verwendung eines Katalysatorsystems bestehend aus einem Titansilikat und Mineralsäure.

Als Alkoxylierungskatalysatoren in der Katalysatormischung kommen vorzugsweise saure Katalysatoren in Form von festen sauren Heterogenkatalysatoren und feste basische heterogene Katalysatoren in Betracht.

Feste Alkoxylierungskatalysatoren sind solche, die sich nicht im Reaktionsmedium lösen und bei der Umsetzung als feste Phase (als Heterogenkatalysatoren) vorliegen.

Bevorzugt werden feste saure Heterogenkatalysatoren auf Basis von geträgerten Mineralsäuren, polymeren sauren Ionenaustauscherharzen, Kompositen aus sauren Ionenaustauscherharzen in anorganischen Materialien, sauren Metalloxiden oder sauren Zeolithen. Beispiele für solche Heterogenkatalysatoren sind saure Schichtsilikate vom K10-Typ, saure Metalloxide, wie sie von Arata in Appl. Catalysis A: General 146 (1996), 3-32, beschrieben werden, und saure Zeolithe vom Strukturtyp MFI (z.B. Zeolith H-ZSM-5), MEL, MFI/MEL, BEA (z.B. H-B-β-Zeolith), MOR, FER, NES, ERI, OFF, MAZ, FAU, TON, CHA, RUT, BOG, LTA, NON, MTN, HEU, AFI, MTW, DOH, EUO, MTT, RHO, CAN, LTL, GIS, GME, VFI, EMT, DDR, SGT, CON, ZON oder MFS.

Bevorzugt werden weiterhin feste basische heterogene Katalysatoren auf Basis von Alkali- oder Erdalkalimetalloxiden oder -hydroxiden, geträgerten Basen, polymeren basischen Ionenaustauscherharzen, Dentrimeraminen, Talciten oder Hydrotalciten.

Die beschriebene Katalysatormischung enthält in der Regel 1 bis 99 Gew.-Teile Epoxidierungskatalysatoren und 99 bis 1 Gew.-Teile Alkoxylierungskatalysatoren, wenn letztere in fester Form, d.h. als Heterogenkatalysatoren, vorliegen. Bevorzugte Bereiche für die Anteile dieser beiden Katalysatorsorten sind 5 bis 95 Gew.-Teile : 95 bis 5 Gew.-Teile und insbesondere 20 bis 80 Gew.-Teile : 80 bis 20 Gew.-Teile. Die beschriebene Katalysatormischung kann darüber hinaus noch weitere übliche Hilfsstoffe enthalten. Liegen freie Mineralsäuren als Alkoxylierungskatalysatoren vor, beträgt der Anteil von Epoxidierungskatalysatoren zu Alkoxylierungskatalysatoren normalerweise 90 bis 99,999 Gew.-Teile : 10 bis 0,001 Gew.-Teile, insbesondere 99 bis 99,99 Gew.-Teile : 1 bis 0,01 Gew.-Teile.

Da die beschriebene Mischung aus den festen Epoxidierungskatalysatoren und den festen Alkoxylierungskatalysatoren neu ist, ist auch Gegenstand der vorliegenden Erfindung eine Katalysatormischung zur einstufigen Epoxidierung und Alkoxylierung von Olefinen aus 1 bis 99 Gew.-Teilen Epoxidierungskatalysatoren und 99 bis 1 Gew.-Teilen festen Alkoxylierungskatalysatoren.

Als Epoxidierungsreagenz für das erfindungsgemäße Verfahren eignen sich vor allem wäßriges Wasserstoffperoxid oder eine Wasserstoff-Sauerstoff-Mischung, der Einsatz von Wasserstoff-Sauerstoff-Mischungen zur Epoxidierung ist beispielsweise in der DE-A 44 25 672 beschrieben. Als Epoxidierungsreagenz sind jedoch auch organische Persäuren oder Hydroperoxide geeignet.

Als hydroxylgruppenhaltige organische Verbindungen kommen prinzipiell jegliche Mono- und Polyhydroxyverbindungen mit ausreichender O-H-Acidität in Betracht. Bevorzugt werden lineare oder verzweigte C₁- bis C₂₀-Alkanole, C₅- bis C₈-Cycloalkanole und C₇- bis C₂₀-Arylalkanole. Beispiele für solche Alkohole sind Methanol, Ethanol, Propanol, iso-Propanol, Butanol, iso-Butanol, sec.-Butanol, tert.-Butanol, Pentanol, iso-Pentanol, sec.-Pentanol, tert.-Pentanol, neo-Pentanol, Hexanol, Heptanol, Octanol, 2-Ethylhexanol, Nonanol, iso-Nonanol, Decanol, Undecanol, Dodecanol, Tridecanol, iso-Tridecanol, Tetradecanol, Pentadecanol, Hexadecanol, Heptadecanol, Octadecanol, Eicosanol, Cyclopentanol, Cyclohexanol, Cycloheptanol, Cyclooctanol, Benzylalkohol, 2-Phenylethanol, 3-Phenylpropanol und 4-Phenylbutanol. Es können auch Mischungen der genannten Alkohole eingesetzt werden. Ganz besonders bevorzugt werden C₁- bis C₈-Alkanole.

Die hydroxylgruppenhaltigen organischen Verbindungen werden, bezogen auf die Äquivalente an ethylenisch ungesättigten Doppelbindungen des Olefins, in stöchiometrischer Menge oder im Überschuß, meist zusätzlich als Lösungsmittel, eingesetzt. Reagieren die hydroxylgruppenhaltigen Verbindungen auch mit zusätzlichen funktionellen Gruppen in den Olefinen, so ist entsprechend mehr einzusetzen.

Das eingesetzte Olefin kann eine beliebige organische Verbindung sein, die mindestens eine ethylenisch ungesättigte Doppelbindung enthält. Sie kann aliphatischer, aromatischer oder cycloaliphatischer Natur sein, sie kann aus einer linearen oder einer verzweigten Struktur bestehen. Vorzugsweise enthält das Olefin 2 bis 30 C-Atome. Mehr als eine ethylenisch ungesättigte Doppelbindung kann vorhanden sein, so etwa in Dienen oder Trienen. Das Olefin kann zusätzlich funktionelle Gruppen wie Halogenatome, Carboxylgruppen, Carbonesterfunktionen, Hydroxylgruppen, Etherbrücken, Sulfidbrücken, Carbonylfunktionen, Cyanogruppen, Nitrogruppen oder Aminogruppen enthalten.

Typische Beispiele für derartige Olefine sind Ethylen, Propen, 1-Buten, cis- und trans-2-Buten, 1,3-Butadien, Pentene, Isopren, Hexene, Octene, Nonene, Decene, Undecene, Dodecene, Cyclopenten, Cyclohexen, Dicyclopentadien, Methylencyclopropan, Vinylcyclohexan, Vinylcyclohexen, Allylchlorid, Acrylsäure, Methacrylsäure, Crotonsäure, Vinylessigsäure, Allylalkohol, Alkylacrylate, Alkylmethacrylate, Ölsäure, Linolsäure, Linolensäure, Ester und Glyceride derartiger ungesättigter Fettsäuren, Styrol, α-Methylstyrol, Divinylbenzol, Inden und Stilben. Auch Mischungen der genannten Olefine können in dem erfindungsgemäßen Verfahren eingesetzt werden.

Das erfindungsgemäße Verfahren eignet sich in besonderem Maße zur Herstellung von Glykolmonoethern aus linearen oder verzweigten C₂-bis C₅-Alkenen, insbesondere Propen.

Die nach dem erfindungsgemäßen Verfahren hergestellten Glykolmonoether weisen die Struktureinheit 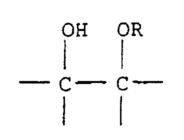 auf, wobei R der Rest der eingesetzten hydroxylgruppenhaltigen organischen Verbindung ist. Meist liegen die Glykolmonoether als Isomerengemische vor, bei denen die OH- und die OR-Gruppe jeweils vertauscht sind.

Die Reaktionsbedingungen für das erfindungsgemäße Verfahren in Bezug auf Temperatur, Druck, Zugabemodus der Einsatzstoffe und Umsetzungsdauer schwanken in Abhängigkeit von den Strukturen der Einsatzstoffe. Als allgemeine Regel kann aufgestellt werden, daß mit steigender Kettenlänge oder zunehmender Molekülgröße von eingesetzten Olefinen und eingesetzten hydroxylgruppenhaltigen organischen Verbindungen die Reaktivität des Systems abnimmt und die Reaktionsbedingungen somit verschärft werden müssen.

Typische Reaktionsbedingungen für die Umsetzung von linearen oder verzweigten C₂- bis C₅-Alkenen, welche unter Normalbedingungen meist gasförmig sind, mit wäßrigem H₂O₂ in Anwesenheit von C₁- bis C₈-Alkanolen, die normalerweise hierbei in überschüssiger Menge vorliegen, sind folgende: Temperatur -30°C bis +80°C, insbesondere - 10°C bis +50°C, unter Eigendruck bei Umsetzungstemperatur, Umsetzungsdauer 1 bis 10 Stunden.

Das erfindungsgemäße Verfahren läßt sich im Labormaßstab und im großtechnischen Maßstab durchführen. Man kann dabei diskontinuierlich oder kontinuierlich arbeiten. In Bezug auf den Kontakt der Reaktionspartner mit der Katalysatormischung bietet sich sowohl eine Slurry- als auch eine Festbettfahrweise an. Die Umsetzung kann in gasförmiger, flüssiger oder überkritischer Phase erfolgen, wobei die flüssige Phase bevorzugt wird.

Vorteilhaft ist ferner, daß bei Verwendung von heterogenen Epoxidierungs- und Alkoxylierungskatalysatoren desaktivierte Katalysatoren durch Wäsche mit dem zur Reaktion einzusetzenden Alkohol oder thermisch oxidativ regenerierbar sind.

Das erfindungsgemäße Verfahren liefert in vielen Fällen praktisch vollständige Umsetzungen der Olefine zu den Glykolmonoethern. Sollten noch merkliche Mengen an intermediär gebildeten Epoxiden im Endprodukt vorliegen, können diese meist durch einfache Methoden vollständig entfernt werden, beispielsweise durch Abdestillieren oder Ausgasen (bei leicht flüchtigen Epoxiden wie Propylenoxid).

Die nachfolgenden Beispiele sollen stellvertretend das erfindungsgemäße Verfahren erläutern, ohne daß dadurch eine Einschränkung zu verstehen wäre. Die Herstellungsbedingungen, Umsätze und Ausbeuten wurden nicht optimiert.

### Beispiele

### Beispiel 1: Herstellung eines Epoxidierungskatalysators

In einem Vierhalskolben (2 l Inhalt) wurden 455 g Tetraethylorthosilikat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Abschließend versetzte man mit 800 g einer 20 gew.-%igen Tetrapropylammoniumhydroxid-Lösung (Alkaligehalt < 10 ppm) und rührte noch eine Stunde nach. Bei 90°C bis 100°C wurde das aus der Hydrolyse gebildete Alkoholgemisch (ca. 450 g) abdestilliert. Man füllte mit 1,5 l deionisiertem Wasser auf und gab das mittlerweile leicht opaque Sol in einen 2,5 l fassenden Rührautoklaven aus Edelstahl.

Mit einer Heizrate von 3°C/min wurde der verschlossene Autoklav (Ankerrührer, 200 U/min) auf eine Reaktionstemperatur von 175°C gebracht. Nach 92 Stunden wurde die Reaktion beendet. Das erkaltete Reaktionsgemisch (weiße Suspension) wurde abzentrifugiert und mehrfach mit Wasser neutralgewaschen. Der erhaltene Feststoff wurde bei 110°C innerhalb von 24 Stunden getrocknet (Auswaage 149 g). Abschließend wurde unter Luft bei 550°C in 5 Stunden das im Zeolithen noch verbliebene Templat abgebrannt (Kalzinierungsverlust: 14 Gew.-%).

Das reinweiße Produkt hatte nach naßchemischer Analyse einen Ti-Gehalt von 1,5 Gew.-% und einen Gehalt an Restalkali unterhalb 100 ppm. Die Ausbeute auf eingesetztes SiO₂ betrug 97 %. Die Kristallite hatten eine Größe von 0,05 bis 0,25 µm und das Produkt zeigte im Infrarotspektrum eine typische Bande bei ca. 960 cm⁻¹.

### Beispiel 2: Herstellung eines Alkoxylierungskatalysators

In einem Becherglas wurden 60,0 g Borsäure in einer aus 343,8 g Tetraethylammoniumhydroxid (40 Gew.-% in Wasser) und 206,2 g deionisiertem Wasser hergestellten Lösung aufgelöst. Diese Lösung gab man in einen 2,5 l fassenden Rührautoklaven aus Edelstahl. Dazu gab man unter Rühren 550,0 g kolloidales Kieselsol (Ludox® AS40).

Der Ansatz wurde bei 150°C für die Dauer von 216 Stunden zur Kristallisation gebracht, abgetrennt, mit deionisiertem Wasser nachgewaschen und bei 120°C für 24 Stunden getrocknet. Die Auswaage betrug 279 g. Abschließend wurde das Produkt bei 500°C unter Luft für die Dauer von 5 Stunden zum H-B-β-Zeolith kalziniert.

### Beispiel 3: Einstufige Herstellung eines Glykolmonoethers aus Propen und Methanol

In einem 250 ml Glasautoklaven wurden 45 ml Methanol und 1,5 g Titansilikalitpulver aus Beispiel 1 sowie 1,5 g Zeolith H-ZSM-5 eingefüllt und die Suspension wurde mit einem Magnetrührer gerührt. Der verschlossene Glasautoklav wurde danach auf -30°C abgekühlt und 20,7 g Propen wurden aufgepreßt. Danach wurde der Glasautoklav auf 0°C erwärmt und 30 g 30 gew.-%ige Wasserstoffperoxidlösung wurden zudosiert. Die Reaktionsmischung wurde 5 h bei 0°C unter Eigendruck gerührt. Danach wurde der Katalysator abzentrifugiert und die Lösung gaschromatographisch untersucht. Der Gehalt an Propylenoxid betrug 9,7 Gew.-%, der Gehalt an Methoxypropanolen 8,2 Gew.-%

### Beispiel 4: Einstufige Herstellung eines Glykolmonoethers aus Propen und Ethanol

In einen 250 ml Glasautoklaven wurden 45 ml Ethanol und 1,5 g Titansilikalitpulver aus Beispiel 1 sowie 1,5 g Zeolith H-ZSM-5 eingefüllt und die Suspension wurde mit einem Magnetrührer gerührt. Der verschlossene Glasautoklav wurde danach auf -30°C abgekühlt und 20,7 g Propen wurden aufgepreßt. Danach wurde der Glasautoklav auf 0°C erwärmt und 30 g 30 gew.-%ige Wasserstoffperoxidlösung wurden zudosiert. Die Reaktionsmischung wurde 5 h bei 0°C unter Eigendruck gerührt. Danach wurde der Katalysator abzentrifugiert und die Lösung gaschromatographisch untersucht. Der Gehalt an Propylenoxid betrug 4,5 Gew.-%, der Gehalt an Ethoxypropanolen 2,2 Gew.-%.

### Beispiel 5: Einstufige Herstellung eines Glykolmonoethers aus Propen und Butanol

In einen 250 ml Glasautoklaven wurden 45 ml Butanol und 1,5 ml Titansilikalitpulver aus Beispiel 1 sowie 1,5 g H-B-β-Zeolith aus Beispiel 2 eingefüllt und die Suspension wurde mit einem Magnetrührer gerührt. Der verschlossene Glasautoklav wurde danach auf -30°C abgekühlt und 20,7 g Propen wurden aufgepreßt. Danach wurde der Glasautoklav auf 0°C erwärmt und 30 g 30 gew.-%ige Wasserstoffperoxidlösung wurden zudosiert. Die Reaktionsmischung wurde 5 h bei 0°C unter Eigendruck gerührt. Danach wurde der Katalysator abzentrifugiert und die Lösung gaschromatographisch untersucht. Der Gehalt an Propylenoxid betrug 0,3 Gew.-%, der Gehalt an Butoxypropanolen 3,8 Gew.-%.

### Beispiel 6: Einstufige Herstellung eines Glykolmonoethers aus Propen und Ethanol

In einen 250 ml Glasautoklaven wurden 45 ml Ethanol und 1,5 ml Titansilikalitpulver aus Beispiel 1 sowie 1,5 g polymerer, saurer Kationenaustauscher (Lewatit®, Fa. Bayer) eingefüllt und die Suspension wurde mit einem Magnetrührer gerührt. Der verschlossene Glasautoklav wurde danach auf -30°C abgekühlt und 20,7 g Propen wurden aufgepreßt. Danach wurde der Glasautoklav auf 0°C erwärmt und 30 g 30 gew.-%ige wasserstoffperoxidlösung wurden zudosiert. Die Reaktionsmischung wurde 5 h bei 0°C unter Eigendruck gerührt. Danach wurde der Katalysator abzentrifugiert und die Lösung gaschromatographisch untersucht. Der Gehalt an Propylenoxid betrug 4,5 Gew.-%, der Gehalt an Ethoxypropanolen 2,2 Gew.-%.

### Vergleichsbeispiel A: Einstufige Herstellung eines Glykolmonoethers aus Propen und Methanol

In einen 250 ml Glasautoklaven wurden 45 ml Methanol und 1,5 g Titansilikalitpulver aus Beispiel 1 eingefüllt und die Suspension wurde mit einem Magnetrührer gerührt. Der verschlossene Glasautoklav wurde danach auf -30°C abgekühlt und 5,8 g Propen wurden aufgepreßt. Danach wurde der Glasautoklav auf 0°C erwärmt und 32 g 30 gew.-%ige Wasserstoffperoxidlösung wurden zudosiert. Die Reaktionsmischung wurde 2 h bei 0°C unter Eigendruck gerührt. Danach wurde der Katalysator abzentrifugiert und die Lösung gaschromatographisch untersucht. Der Gehalt an Propylenoxid betrug 8,65 Gew.-%, der Gehalt an Methoxy-2-propanol 0,04 Gew.-% und der Gehalt an Methoxy-3-propanol 0,09 Gew.-%.

## Patentansprüche

1. Verfahren zur Herstellung von Glykolmonoethern aus Olefinen, **dadurch gekennzeichnet, daß** man die Olefine mit einem Epoxidierungsreagenz bei gleichzeitiger Anwesenheit von hydroxylgruppenhaltigen organischen Verbindungen an einer Mischung aus Epoxidierungskatalysatoren und festen heterogenen Alkoxylierungskatalysatoren umsetzt.

2. Verfahren nach Anspruch 1, **dadurch gekennzeichnet, daß** man als Epoxidierungskatalysatoren in der Katalysatormischung titanhaltige Silikate oder titan-, vanadium-, germanium-oder zinnhaltige Zeolithe, insbesondere Titan- oder Vanadiumsilikalite mit Zeolith-Struktur mit röntgenographischer Zuordnung zur MFI-, MEL-, BEA-, MTW-, TON-, FER- oder MFI/MEL-Mischstruktur, einsetzt.

3. Verfahren nach Anspruch 1 oder 2, **dadurch gekennzeichnet, daß** man als feste Alkoxylierungskatalysatoren in der Katalysatormischung saure Katalysatoren in Form von festen sauren Heterogenkatalysatoren oder feste basische heterogene Katalysatoren einsetzt.

4. Verfahren nach Anspruch 3, **dadurch gekennzeichnet, daß** man als feste saure Alkoxylierungskatalysatoren in der Katalysatormischung feste saure Heterogenkatalysatoren auf Basis von geträgerten Mineralsäuren, polymeren sauren Ionenaustauscherharzen, Kompositen aus sauren Ionenaustauscherharzen in anorganischen Materialien, sauren Metalloxiden oder sauren zeolithen einsetzt.

5. Verfahren nach Anspruch 3, **dadurch gekennzeichnet, daß** man als feste basische Alkoxylierungskatalysatoren in der Katalysatormischung feste basische heterogene Katalysatoren auf Basis von Alkali- oder Erdalkalimetalloxiden oder -hydroxiden, geträgerten Basen, polymeren basischen Ionenaustauscherharzen, Dendrimeraminen, Talciten oder Hydrotalciten einsatzt.

6. Verfahren nach den Ansprüchen 1 bis 5, **dadurch gekennzeichnet, daß** man als Epoxidierungsreagenz wäßriges Wasserstoffperoxid oder eine Wasserstoff-Sauerstoff -Mischung einsetzt.

7. Verfahren nach den Ansprüchen 1 bis 6, **dadurch gekennzeichnet, daß** man als hydroxylgruppenhaltige organische Verbindungen lineare oder verzweigte C₁- bis C₂₀-Alkanole, C₅-bis C₈-Cycloalkanole oder C₇- bis C₂₀-Arylalkanole einsetzt.

8. Verfahren nach den Ansprüchen 1 bis 7, **dadurch gekennzeichnet, daß** man als Olefine solche mit 2 bis 30 C-Atomen mit einer oder mehreren ethylenisch ungesättigten Doppelbindungen einsetzt.

9. Verfahren nach Anspruch 8, **dadurch gekennzeichnet, daß** man als Olefine lineare oder verzweigte C₂- bis C₅-Alkene, insbesondere Propen, einsetzt.

10. Katalysatormischung zur einstufigen Epoxidierung und Alkoxylierung von Olefinen aus 1 bis 99 Gew.-Teilen Epoxidierungskatalysatoren und 99 bis 1 Gew.-Teilen festen Alkoxylierungskatalysatoren.

## Claims

1. A process for preparing glycol monoethers from olefins, which comprises reacting the olefins with an epoxidizing reagent in the simultaneous presence of hydroxyl-containing organic compounds over a mixture of epoxidation catalysts and solid heterogeneous alkoxylation catalysts.

2. A process as claimed in claim 1, wherein the epoxidation catalysts used in the catalyst mixture are titanium-containing silicates or titanium-, vanadium-, germanium- or tin-containing zeolites, in particular titanium or vanadium silicalites having a zeolite structure assigned by X-ray diffraction to the MFI, MEL, BEA, MTW, TON, FER or MFI/MEL mixed structure.

3. A process as claimed in claim 1 or 2, wherein the solid alkoxylation catalysts used in the catalyst mixture are acidic catalysts in the form of solid acidic heterogeneous catalysts, or solid basic heterogeneous catalysts.

4. A process as claimed in claim 3, wherein the solid acidic alkoxylation catalysts used in the catalyst mixture are solid heterogeneous catalysts based on supported mineral acids, polymeric acidic ion exchange resins, composites of acidic ion exchange resins in inorganic materials, acidic metal oxides or acidic zeolites.

5. A process as claimed in claim 3, wherein the solid basic alkoxylation catalysts used in the catalyst mixture are solid basic heterogeneous catalysts based on alkali metal or alkaline earth metal oxides or hydroxides, supported bases, polymeric basic ion exchange resins, dendrimeric amines, talcites or hydrotalcites.

6. A process as claimed in any of claims 1 to 5, wherein the epoxidizing reagent used is aqueous hydrogen peroxide or a hydrogen/oxygen mixture.

7. A process as claimed in any of claims 1 to 6, wherein the hydroxyl-containing organic compounds used are linear or branched C₁- to C₂₀-alkanols, C₅- to C₈-cycloalkanols or C₇-to C₂₀-arylalkanols.

8. A process as claimed in any of claims 1 to 7, wherein the olefins used are those having from 2 to 30 carbon atoms and one or more ethylenically unsaturated double bonds.

9. A process as claimed in claim 8, wherein the olefins used are linear or branched C₂- to C₅-alkenes, in particular propene.

10. A catalyst mixture for the single-stage epoxidation and alkoxylation of olefins, consisting of from 1 to 99 parts by weight of epoxidation catalysts and from 99 to 1 parts by weight of solid alkoxylation catalyts.

## Revendications

1. Procédé de préparation de monoéthers glycoliques à partir d'oléfines, **caractérisé en ce que** l'on met les oléfines à réagir avec un réactif d'époxydation en présence simultanée de composés organiques contenant des groupes hydroxyle sur un mélange de catalyseurs d'époxydation et de catalyseurs d'alcoxylation hétérogènes solides.

2. Procédé selon la revendication 1, **caractérisé en ce que** l'on met en oeuvre en tant que catalyseurs d'époxydation dans le mélange de catalyseurs des silicates contenant du titane ou des zéolites contenant du titane, du vanadium, du germanium, ou de l'étain, en particulier des silicalites de titane ou de vanadium ayant une structure de zéolite avec une classification de structure mixte MFI, MEL, BEA, MTW, TON, FER ou MFI/MEL, obtenue par analyse aux rayons X.

3. Procédé selon la revendication 1 ou 2, **caractérisé en ce que** l'on met en oeuvre en tant que catalyseurs d'alcoxylation solides dans le mélange de catalyseurs, des catalyseurs acides sous forme de catalyseurs hétérogènes acides solides ou de catalyseurs hétérogènes basiques solides.

4. Procédé selon la revendication 3, **caractérisé en ce que** l'on met en oeuvre en tant que catalyseurs d'alcoxylation acides solides dans le mélange de catalyseurs, des catalyseurs hétérogènes acides solides à base d'acides minéraux supportés, de résines acides polymères échangeuses d'ions, de composites constitués de résines acides échangeuses d'ions dans des matériaux inorganiques, d'oxydes métalliques acides ou de zéolites acides.

5. Procédé selon la revendication 3, **caractérisé en ce que** l'on met en oeuvre en tant que catalyseurs d'alcoxylation basiques solides dans le mélange de catalyseurs, des catalyseurs hétérogènes basiques solides à base d'oxydes ou d'hydroxydes de métal alcalin ou alcalino-terreux, de bases supportées, de résines basiques polymères échangeuses d'ions, d'amines dendrimères, de talcites, ou d'hydrotalcites.

6. Procédé selon les revendications 1 à 5, **caractérisé en ce que** l'on met en oeuvre en tant que réactif d'époxydation, du peroxyde d'hydrogène aqueux ou un mélange d'hydrogène/oxygène.

7. Procédé selon les revendications 1 à 6, **caractérisé en ce que** l'on met en oeuvre en tant que composé organique contenant des groupes hydroxyle, des alcanols linéaires ou ramifiés en C₁ à C₂₀, des cycloalcanols en C₅ à C₈, ou des arylalcanols en C₇ à C₂₀.

8. Procédé selon les revendications 1 à 7, **caractérisé en ce que** l'on met en oeuvre en tant qu'oléfines, celles ayant 2 à 30 atomes de C avec une ou plusieurs doubles liaisons à insaturation éthylénique.

9. Procédé selon la revendication 8, **caractérisé en ce que** l'on met en oeuvre en tant qu'oléfines, des alcènes linéaires ou ramifiés en C₂ à C₅, en particulier le propène.

10. Mélange de catalyseurs pour l'époxydation et l'alcoxylation d'oléfines en une seule étape, constitué de 1 à 99 parties en poids de catalyseurs d'époxydation et 99 à 1 parties en poids de catalyseurs d'alcoxylation solides.
